# EUROPEAN PATENT APPLICATION

(11) **EP 1 769 789 A2**
(43) Date of publication of application: **04.04.2007**
(21) Application number: 06121491.2
(22) Date of filing: 29.09.2006
(51) Int. Cl.: A61K 8/97, A61K 8/89, A61K 31/165

(54) **Topical composition comprising capsaicin**

(30) Priority: 30.09.2005 IT MI20051848
(71) Applicant: Sirton Medicare SPA, 22079 Villa Guardia CO (IT)
(72) Inventor: Ferro, Laura Iris, 20121, Milano (IT); Virgili, Valerio, 28040, Paruzzaro (Novara) (IT); Beretti, Constanzo, 24100, Brescia (IT)
(74) Representative: Pistolesi, Roberto

(57) **Abstract**

The present invention relates to a composition for topical use containing capsaicin and silicone for the treatment of dermatoses with a pruriginous component together with protection from exogenous agents.

## Description

Capsaicin is the active ingredient contained in the dried ripe fruit of various species of Capiscum (Solanaceae) and the most important varieties are Capiscum annum (red pepper whose powder is given the name Paprika) and Capiscum frutescens (strong red pepper or Cayenne pepper). Capsaicin is also produced synthetically while maintaining the same properties of the extracted product.

The topical action of capsaicin is well known in the literature: it stimulates the type 1 vanilloid receptors (VR1) present in the nociceptors of the skin (non-myelinated C fibres) which carry the nerve signals to the dorsal ganglions of the central nervous system, bringing about the release of neurotransmitters, such as substance

P, somatostatin and calcitonin gene-related peptide (CGRP). These neurotransmitters in turn cause the sensation of pain.

The protopathic sensation of itching, while being distinct from that of pain, also travels through the same nerve fibres: it is transported along the C fibres and is mediated by the release in the central nervous system of substance P.

The pain-killing and anti-pruriginous capabilities of capsaicin for topical use are not manifested immediately but develop over time because, after an initial increase in the perception of pain and burning, caused by the release of neurotransmitters in the central nervous system, a state of reduced sensitivity sets in and, after repeated applications, prolonged desensitisation of the treated area, caused by the depletion of the neurotransmitters, is obtained.

Numerous exogenous causes may bring about the onset of contact dermatosis with a pruriginous component: the skin undergoes attack because, for example, of prolonged contact with water, contact with water-based or oil-based substances, contact with metals, pollution, and changes in temperature, to mention just a few of the major causes of contact dermatitis documented.

Of the various existing methods for the physical protection of the skin, the role played by the so-called barrier creams, which are generally based on silicone, is important, being formulated in such a manner as to create a protective film on the skin, and thus to defend it from external attack. Such creams, while not representing a therapeutic product for the cure or prevention of a skin disease or a symptom thereof, combine with the physical protection the capacity to maintain the hydration of the stratum corneum.

The qualitative-quantitative formulation of the present invention comprises capsaicin in a range of from 0.05% to 0.15% by weight with respect to the total weight of the composition, polydimethylsiloxane (commercially available from Wacker under the name Silfar® 350) at from 2% to 4% by weight with respect to the total weight of the composition, and excipients and/or coadjuvants.

This new formulation for topical use has the protective characteristics of film-forming barrier creams based on silicone, together with a skin-softening action, owing to its formulation as a soft white cream of the oil-in-water emulsion type. Surprisingly, the protective action of the silicone barrier cream, in combination with its emollient properties and the desensitising properties of capsaicin, has led to a significant decrease in the localised itching of the skin caused by contact dermatitis brought about by exogenous agents.

The present invention therefore relates to topical (pharmaceutical and/or "medical device") compositions essentially containing (or preferably consisting of) from 0.05% to 0.15% of capsaicin, from 2% to 4% of polydimethylsiloxane, from 5% to 7% of a non-ionic emulsifier, from 2.50% to 3.50% of cetyl alcohol, from 5% to 7% of isostearyl isostearate, from 2.50% to 3.50% of propylene glycol, from 3% to 4% of glycerol, from 0.60% to 1% of a solubilising agent, from 0.05% to 0.15% of allantoin, from 0.05% to 0.15% of disodium EDTA, from 0.50% to 0.90% of an antimicrobial agent and water (to make up to 100%), where all of the percentages are expressed by weight with respect to the total weight of the composition.

Particularly preferred are topical compositions essentially containing (or consisting of) from 0.06% to 0.10% of capsaicin, from 2.50% to 3.50% of polydimethylsiloxane, from 5.50% to 6.50% of a non-ionic emulsifier, from 2.80% to 3.30% of cetyl alcohol, from 5.80% to 6.20% of isostearyl isostearate, from 2.70% to 3.20% of propylene glycol, from 3.30% to 3.80% of glycerol, from 0.70% to 0.90% of a solubilising agent, from 0.08% to 0.12% of allantoin, from 0.08% to 0.12% of disodium EDTA, from 0.60% to 0.80% of an antimicrobial agent and water (to make up to 100%), where all of the percentages are expressed by weight with respect to the total weight of the composition.

The preferred non-ionic emulsifier is commercially available from Gattefosse under the name Emulium delta and consists of a mixture of ethoxylated fatty alcohols and stearic esters of glycerol and polyethylene glycol; the solubilising agent, Solprogress, which is commercially available from Prodotti Gianni, is a mixture of polyoxyethylenated hydrogenated castor oil and tridecyl polyoxyethylene ether, while the preferred antimicrobial agent is constituted by Sepicide HB, which is a mixture of methyl, ethyl, propyl and butyl parahydroxybenzoic esters, in association with phenoxyethyl alcohol, commercially available from Seppic.

According to a preferred embodiment of the following invention, the preferred qualitative-quantitative composition of the present invention is indicated in Table 1.

**Table 1**

| Ingredient | % by weight with respect to the total weight of the composition | Specific functions and components |
|---|---|---|
| Silfar® 350 | 3.00 | Silicone base (polydimethylsiloxane) |
| Capsaicin 95% | 0.079 | Active component (percentage equal to a theoretical content of 0.075% of capsaicin) |
| Emulium delta | 6.00 | Non-ionic emulsifier (mixture of ethoxylated fatty alcohols and stearic esters of glycerol and polyethylene |
| | | glycol) |
| Nacol 1618 | 3.00 | Cetyl alcohol |
| Isostearyl isostearate | 6.00 | |
| Propylene glycol | 3.00 | |
| Vegetable glycerol | 3.50 | Hydrating agent |
| Solprogress | 0.80 | Solubiliser (polyoxyethylenated hydrogenated castor oil, tridecyl polyoxyethylene ether) |
| Allantoin | 0.10 | Coadjuvant |
| Disodium EDTA | 0.10 | Synergistic agent |
| Sepicide HB | 0.70 | Antimicrobial preservative (mixture of methyl, ethyl, propyl and butyl parahydroxybenzoic esters, in association with phenoxyethyl alcohol) |
| Water | q.s. | 73.721% + extra for working losses |

The preparation of the preferred composition of the present invention consists in the preparation of an aqueous phase A, comprising water of osmosis, allantoin, disodium EDTA, propylene glycol and vegetable glycerol, and a fatty phase B, comprising Emulium delta, Nacol 1618, isostearyl isostearate and Silfar 350, and in their combination at a temperature of from 70°C to 75°C. The mixture is then cooled to a temperature of less than 50°C and is mixed with phase C, which is constituted by 95% capsaicin, ethyl alcohol and by Solprogress 70. The ethyl alcohol, the solubuliser for the 95% capsaicin and in a ratio of 2:1 with the capsaicin, evaporates almost completely by heating. The preservative, Sepicide HB, is then added to the resulting mixture and, after cooling and analytical monitoring of the emulsion obtained, the latter is distributed in aluminium tubes.

The present invention relates also to the use of capsaicin, dimethylsiloxane and excipients and/or coadjuvants in the quantities indicated above, for the preparation of a topical medicament for the treatment of pruritis induced by contact dermatosis together with protection from exogenous agents.

Particularly preferred is the use of capsaicin and dimethylsiloxane for the preparation of compositions comprising capsaicin in a range of from 0.05% to 0.15%, polydimethylsiloxane at from 2% to 4%, from 5% to 7% of a non-ionic emulsifier, from 2.50% to 3.50% of cetyl alcohol, from 5% to 7% of isostearyl isostearate, from 2.50% to 3.50% of propylene glycol, from 3% to 4% of glycerol, from 0.60% to 1% of a solubilising agent, from 0.05% to 0.15% of allantoin, from 0.05% to 0.15% of disodium EDTA, from 0.50% to 0.90% of an antimicrobial agent and water (to make up to 100%), where all of the percentages are expressed by weight with respect to the total weight of the composition.

Particularly preferred is the use of capsaicin and dimethylsiloxane for the preparation of topical compositions essentially containing (or consisting of) from 0.06% to 0.10% of capsaicin, from 2.50% to 3.50% of polydimethylsiloxane, from 5.50% to 6.50% of a non-ionic emulsifier, from 2.80% to 3.30% of cetyl alcohol, from 5.80% to 6.20% of isostearyl isostearate, from 2.70% to 3.20% of propylene glycol, from 3.30% to 3.80% of glycerol, from 0.70% to 0.90% of a solubilising agent, from 0.08% to 0.12% of allantoin, from 0.08% to 0.12% of disodium EDTA, from 0.60% to 0.80% of an antimicrobial agent and water (to make up to 100%), where all of the percentages are expressed by weight with respect to the total weight of the composition.

## Claims

1. A topical composition in the form of an oil-in-water emulsion comprising capsaicin in an amount of from 0.05% to 0.15% by weight with respect to the total weight of the composition and polydimethylsiloxane in an amount of from 2% to 4% by weight with respect to the total weight of the composition, together with excipients and/or coadjuvants.

2. A topical composition according to claim 1, comprising capsaicin in an amount of from 0.06% to 0.10% by weight with respect to the total weight of the composition and/or polydimethylsiloxane in an amount of from 2.50% to 3.50% by weight with respect to the total weight of the composition.

3. A topical composition according to any one of the preceding claims, comprising from 5% to 7% by weight, and preferably from 5.50% to 6.50%, of a non-ionic emulsifier.

4. A topical composition according to any one of the preceding claims, comprising from 2.50% to 3.50% by weight, and preferably from 2.80% to 3.30% of cetyl alcohol.

5. A topical composition according to any one of the preceding claims, comprising from 5% to 7% by weight, and preferably from 5.80% to 6.20% of isostearyl isostearate.

6. A topical composition according to any one of the preceding claims, comprising from 2.50% to 3.50% by weight, and preferably from 2.70% to 3.20%, of propylene glycol.

7. A topical composition according to any one of the preceding claims, comprising from 3% to 4% by weight, and preferably from 3.30% to 3.80%, of glycerol.

8. A topical composition according to any one of the preceding claims, comprising from 0.60% to 1% by weight, and preferably from 0.70% to 0.90%, of a solubilising agent.

9. A topical composition according to any one of the preceding claims, comprising from 0.05% to 0.15% by weight, and preferably from 0.08% to 0.12%, of allantoin.

10. A topical composition according to any one of the preceding claims, comprising from 0.05% to 0.15% by weight, and preferably from 0.08% to 0.12%, of disodium EDTA.

11. A topical composition according to any one of the preceding claims, comprising from 0.50% to 0.90% by weight, and preferably from 0.60% to 0.80%, of an antimicrobial agent.

12. A topical composition according to any one of the preceding claims, comprising water to make up to 100% by weight of the composition.

13. A topical composition according to claim 1, containing approximately 0.079% of capsaicin, approximately 3% of polydimethylsiloxane, approximately 6% of a non-ionic emulsifier, approximately 3% of cetyl alcohol, approximately 6% of isostearyl isostearate, approximately 3% of propylene glycol, approximately 3.5% of glycerol, approximately 0.80% of a solubilising agent, approximately 0.10% of allantoin, approximately 0.10% of disodium EDTA, approximately 0.70% of an antimicrobial agent and approximately 73.721 % of water, where all of the percentages are expressed by weight with respect to the total weight of the composition.

14. A topical composition according to claim 13, **characterised in that** the capsaicin has a purity of 95%.

15. A topical composition according to the preceding claims, wherein the non-ionic emulsifier is constituted by a mixture of ethoxylated fatty alcohols and of stearic esters of glycerol.

16. A topical composition according to the preceding claims, wherein the solubilising agent comprises a mixture of polyoxyethylenated hydrogenated castor oil and tridecyl polyoxyethylene ether.

17. A topical composition according to the preceding claims, wherein the antimicrobial agent is constituted by a mixture of methyl, ethyl, propyl and butyl parahydroxybenzoic esters in association with phenoxyethyl alcohol.

18. A topical composition according to the preceding claims in the form of a cream.

19. Use of capsaicin in an amount of from 0.05% to 0.15% by weight and dimethylsiloxane in an amount of from 2% to 4% by weight, for the preparation of a topical medicament for the treatment of pruritis induced by contact dermatosis and/or for protection from exogenous agents.
